# EUROPEAN PATENT APPLICATION

(11) **EP 1 207 202 A1**
(43) Date of publication of application: **22.05.2002**
(21) Application number: 01126500.6
(22) Date of filing: 12.11.2001
(51) Int. Cl.: C12N 15/12, C07K 14/47, C12N 5/10, C07K 16/18, C12P 13/00, G01N 33/50

(54) **Nucleic acid molecules encoding a protein interacting with the chemokine receptor CCR5 or other chemokine receptor family members**

(30) Priority: 16.11.2000 EP 00125052
(71) Applicant: Mölling, Karin, Inst. für med. Virologie der Uni. Zürich, 8208 Zürich (CH)
(72) Inventor: Mölling, Karin, 8044 Zürich (CH); Schweneker, Marc, 8005 Zürich (CH)
(74) Representative: VOSSIUS & PARTNER

(57) **Abstract**

Disclosed is a nucleic acid molecule encoding a protein interacting with the carboxy terminus of the chemokine receptor CCR5 or of other chemokine receptor family members as well as the encoded protein. Furthermore, the invention describes expression vectors, host cells, antibodies, antagonists, pharmaceutical compositions and methods for treating HIV infection and progression to AIDS.

## Description

The present invention relates to nucleic acid molecules encoding a protein interacting with the carboxy terminus of the chemokine receptor CCR5 or of other chemokine receptor family members. The invention also relates to the encoded protein. The invention furthermore relates to expression vectors, host cells, an antibody against the described protein, antagonists against the protein as well as to pharmaceutical compositions comprising the described nucleic acid molecule, the protein, the antibody or antagonists and to methods for preventing and/or treating HIV infection and progression to AIDS.

The beta chemokine receptor 5, CCR5, acts as a co-receptor in concert with CD4 to mediate internalization of HIV-1 into T-lymphocytes (Wu et al., Nature 381 (1996), 179-183). While CD4 is fully dispensable for cell entry by some virus strains, interaction between the HIV envelope glycoprotein and the co-receptor plays a primary role. Chemokine receptors are members of the seven-transmembrane domain superfamily of receptors which activate cells by coupling to heterotrimeric G-proteins (Feng et al., Science 272 (1996), 872-877; Deng et al., Nature 381 (1996), 661-666).
A deletion of 32 base pairs in the CCR5 gene (delta 32 CCR5) has been linked to resistance to HIV-1 infection in exposed adults and the delay of disease progression in infected adults (Samson et al., Nature 382 (1996), 722-725). Deletion of 32 nucleotides leads to premature termination of the CCR5 receptor which results in a four transmembrane receptor lacking the carboxyterminal two transmembrane regions and the carboxyterminal cytoplasmic tail. The homozygous deletion of the CCR5 carboxyterminus (ct) leads to long-term nonprogressors (LTNP) and is a useful predictive marker to identify individuals with delayed disease progression who may not require anti-retroviral therapy.

Additional chemokine receptors have been implicated as HIV-1 co-receptors on certain cell types. HIV infected individuals harbor predominantly macrophage-tropic HIV isolates during early stages of infection with CCR5 as co-receptors, but accumulate increasing amounts of T-cell tropic strains just before accelerated T-cell depletion and progression to aids. This correlates with viral adaptation to the T-tropic CXCR4 receptor. The identification of dual tropic HIV strains over the course of infection suggest that such strains are intermediates between macrophage and T-cell tropic populations. The tropic transition occurs from CCR5 as early receptor usage to CXCR4 as late and progression to aids. Heterozygous delta 32 CCR5 deletions also lead to reduced disease progression.
The genetic defect which is found in 1% of caucasions does not lead to any phenotype. The molecular mechanism involves downregulation of the mutant CCR5 receptor, i.e. reduced exposure at the cell membrane. This impairs the entry of the virus and prevents replication and may force the virus into attenuated variants. Binding of the viral envelope protein to the receptor can mimic binding of chemokines, resulting in activation on heterotrimeric G-proteins, Ca2+ fluxing and tyrosine phosphorylation. Such signals may play important roles in viral replication (Littman, Cell 93 (1998), 677).
Although it is known that the CCR5 receptor, and possibly other chemokine receptor family members, are involved in the internalization of HIV into T-lymphocytes, nothing is known so far about other factors interacting with CCR5 or other chemokine receptors and possibly required for the internalization. Such factors could be useful for designing drugs for inhibiting the entry of HIV into cells via interaction with the CCR5 receptor other chemokine receptor family members.

Thus, the technical problem underlying the present invention is to identify factors interacting with the carboxy terminus of the CCR5 receptor or of other chemokine receptor family members.

This technical problem is solved by the provision of the embodiments as characterized in the claims.

Accordingly, the present invention relates to nucleic acid molecules encoding a protein interacting with the carboxy terminus of the chemokine receptor CCR5 or of other chemokine receptor family members selected from the group consisting of:
(a) nucleic acid molecules encoding a protein which comprises the amino acid sequence indicated in Seq ID No: 2;
(b) nucleic acid molecules comprising the nucleotide sequence of the coding region indicated in Seq ID No: 1;
(c) nucleic acid molecules encoding a protein, the amino acid sequence of which has a homology of at least 50% to the amino acid sequence indicated in Seq ID No: 2;
(d) nucleic acid molecules the complementary strand of which hybridizes to a nucleic acid molecule as defined in (a) or (b); and
(e) nucleic acid molecules, the nucleotide sequence of which deviates because of the degeneracy of the genetic code from the sequence of the nucleic acid molecules as defined in any one of (b), (c) or (d).

Consequently, the present invention relates to nucleic acid molecules encoding a protein interacting with the carboxy terminus of the chemokine receptor CCR5 or of other chemokine receptor family members, said molecules preferably encoding a protein comprising the amino acid sequence indicated in SEQ ID No: 2. In the context of the present invention the encoded protein is also referred to as P2.

The present invention is based on the isolation of a nucleic acid molecule encoding a protein that interacts with the chemokine receptor CCR5. The nucleotide sequence is shown in SEQ ID NO: 1 and has a length of 1263 nucleotides. It encodes a protein of 178 amino acid residues. An EST identical to the sequence disclosed in SEQ ID NO:1 is known from hypothetical reading frame of human (Accession no.: AJ 005896). The function of the proteins encoded by this hypothetical reading frame is, however, completely unknown.

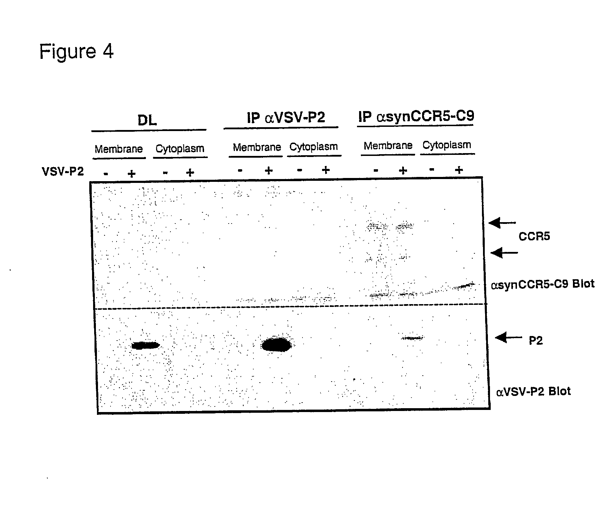

The protein encoded by a nucleic acid molecule of the present invention is capable of interacting with the chemokine receptor CCR5, in particular at a site where no side effects are anticipated, which is the carboxyterminal part deleted, e.g., in LTNP, or with other chemokine receptor family members. The term "carboxy terminus" in this context preferably means the last 20 C-terminal amino acids, more preferably the last 10 C-terminal amino acids. Moreover, said carboxy terminus preferably has a C-terminal leucine, valine or isoleucine residue. The term "interact" in this context means that the protein is able to bind to the carboxy terminus of the CCR5 or of other chemokine receptor family members, in particular when tested in a yeast two-hybrid screening assay. This assay is described in detail in Example 1. Preferably, the protein is able to bind to the carboxy terminus of the CCR5 or of other chemokine receptor family members when tested in the two-hybrid screening assay as described in Example 1.
The term "CCR5" referred to herein means a chemokine receptor of the seven-transmembrane domain superfamily of receptors which activate cells by coupling to heterotrimeric G-proteins (Feng et al., Science 272 (1996), 872-877; Deng et al., Nature 381 (1996), 661-666). In particular, this receptor is a beta chemokine receptor and is described, e.g., in Samson et al. (Biochemistry 35 (1996), 3362-3367) and in Raport et al. (J. Biol. Chem. 271 (1996), 17161-17166). It is characterized, e.g., by the property that it acts as a co-receptor in concert with CD4 to mediate internalization of HIV into Tlymphozytes (Wu et al., Nature 381 (1996), 179-183). The nucleotide and amino acid sequence of a CCR5 receptor, which is preferably meant when reference is made to a CCR5 receptor, is disclosed, e.g. in GenBank Acc. No. X91492.
The CCR5 receptor is also characterized in that it mediates a chemotactic response such as cell migration in response to chemoattractans like MIP-1beta and RANTES (Raport et al., J. Biol. Chem. 271 (1996), 17161-17166).
The ability of the encoded protein to bind to CCR5 or other chemokine receptor family members can also be tested by co-immunoprecipitation as, e.g., described in Example 4 and in the legend to Figure 4.
The term "other chemokine receptor family members" in the context of the present invention means proteins which are classified as chemokine receptors based on their ability to bind to CXC and CC chemokines, also designated as α and β chemokines, respectively, as described, e.g. in Murphy et al., International Union of Pharmacology XXII, Nomenclature for chemokine receptors, Pharmacol. Rev. 52 (2000), 145-176 and which have a C-terminus similar to CCR5 receptor meaning that they have an identity of at least 30%, preferably of at least 40%, more preferably of at least 50% over the last 20 C-terminal amino acids to the CCR5 receptor, and in particular that they are capable of interacting with a hydrophobic pocket and preferably in so far as their carboxy terminus is constituted by a leucine, valine or isoleucine residue. Moreover, such receptors preferably function as viral receptors, in particular for HIV. For example, the term "other chemokine receptor family members" also comprises CCR2 receptors which also have a C-terminal leucine.

The protein encoded by a nucleic acid molecule according to the invention in a preferred embodiment not only interacts with CCR5 or other chemokine receptor family members but also has the capability to interact with the C-terminus of one or more other transmembrane receptors, for example other transmembrane receptors of the seven transmembrane domain superfamily or transmembrane receptors with more than seven, for example 8 to 10, transmembrane domains. Preferred examples are amino acid transporterssuch as the amino acid transporter EAAC1/EAAT3 (Liu et al., Nature 410 (2001), 84-88). EAAC/EAAT receptors belong to the family of excitatory amino acid carriers (or transporters). A tentative consensus sequence of the carboxytermini of CCR5 and of EAAC1/EAAT3 recognized by the protein encoded by a nucleic acid molecule of the invention is:
VGLP .. D . TLIIAVDW . LDR. RT .. NVLGD.. G .. I . E . LS . KEL ....V.... I . ..F.....I...EA.E/D...S....S.....
Functional roles of the family of amino acid transporters are to transport amino acids across plasma membranes. Thus, the protein encoded by a nucleic acid molecule may also be involved in regulation of this transport.

Other preferred examples of transmembrane receptors are those which show sequence homology in their carboxyterminal 80 to 100 amino acids to the carboxyterminus of the CCR5 receptor. Preferably the homology is at least 40%.
Whether the protein encoded by a nucleic acid molecule according to the invention interacts with a given transmembrane receptor or not can be determined by methods known to the person skilled in the art. Assays to determine whether a receptor binds to a protein encoded by a nucleic acid molecule of the invention can be performed, e.g., by two-hybrid screens of the cytoplasmic carboxyterminal 80 to 100 amino acids of the receptor as bait to the protein of the invention as target. This method is described in Example 1. Another possibility is the use of immunoprecipitation assays. In such an assay the receptor in question and the protein encoded by a nucleic acid molecule of the invention are expressed in a suitable host cell. Then either the receptor or the protein is immunoprecipitated and it is determined whether the protein or the receptor, respectively, is co-precipitated. A co-precipitation is indicative of an interaction of the receptor and the protein. In order to determine whether the interaction involves the C-terminus of the transmembrane receptor, mutants of the receptor in which the C-terminus is deleted or mutated can be tested in the same manner as described above or in a two-hybrid system.

Apart from the capability to interact with CCR5 the protein encoded by a nucleic acid molecule according to the invention is furthermore preferably characterized by at least one of the following features:
(a) it does no longer interact with CCR5 or other chemokine receptor family members if in the CCR5 protein or other chemokine receptor family members the very carboxyterminal leucine (in the case of CCR5 L352) is mutated, e.g. if it is mutated to proline;
(b) it requires its carboxy terminal domain, preferably the region corresponding to amino acid residues 139 to 178 of SEQ ID NO:2, to interact with CCR5 or other chemokine receptor family members;
(c) it contains in its carboxy terminal region a motif which is remotely related to the GLGF sequence of the so-called PDZ domain (Schneider et al., Nature Biotech. 17 (1999), 170-175), and which preferably has the sequence SIGL (corresponding to amino acid residues 155-158 of SEQ ID NO:2);
(d) it can be distinguished from known PDZ proteins, i.e. proteins containing a PDZ domain, due to the lack of the 6 β-sheets and 2 α-helices characteristic for PDZ proteins;
(e) it can no longer interact with CCR5 or other chemokine receptor family members if the region containing the motif described in (c), above, is removed, or if the motif described in (c), above, is mutated, e.g. in such a way that the hydrophobic pocket build by this motif is destroyed, for example by changing the amino acid residue corresponding to residue G157 to R (G157R);
(f) computer predictions using the amino acid sequence of the protein indicate that it is a transmembrane protein with two to four transmembrane domains; and
(g) the amino terminus and the carboxy terminus of the protein face the cytoplasmic region of the cell membrane.

Moreover, the present invention relates to nucleic acid molecules which encode a protein which interacts with the chemokine receptor CCR5 or other chemokine receptor family members and the complementary strand of which hybridizes with one of the above-described molecules.
The present invention also relates to nucleic acid molecules which encode a protein, which has a homology, that is to say a sequence identity, of at least 50%, preferably of at least 60%, more preferably of at least 65%, even more preferably of at least 70% and particularly preferred of at least 80%, especially preferred of at least 90% and even more preferred of at least 95% to the entire amino acid sequence as indicated in Seq ID No: 2.
Moreover, the present invention relates to nucleic acid molecules which encode a protein which interacts with the chemokine receptor CCR5 or other chemokine receptor family members and the nucleotide sequence of which has a homology, that is to say a sequence identity, of at least 40%, preferably of at least 50%, more preferably of at least 60%, even more preferably of more than 65%, in particular of at least 70%, especially preferred of at least 80%, in particular of at least 90% and even more preferred of at least 95% when compared to the coding region of the sequence shown in SEQ ID NO:1.
The present invention also relates to nucleic acid molecules, which encode a protein which interacts with the chemokine receptor CCR5 or other chemokine receptor family members and the sequence of which deviates from the nucleotide sequences of the above-described nucleic acid molecules due to the degeneracy of the genetic code.
The invention also relates to nucleic acid molecules comprising a nucleotide sequence which is complementary to the whole or a part of one of the above-mentioned sequences.

In the context of the present invention the term "hybridization" means hybridization under conventional hybridization conditions, preferably under stringent conditions, as for instance described in Sambrook et al., Molecular Cloning, A Laboratory Manual, 2nd edition (1989) Cold Spring Harbor Laboratory Press, Cold Spring Harbor, NY. In an especially preferred embodiment the term "hybridization" means that hybridization occurs under the following conditions:

| | |
|---|---|
| Hybridization buffer: | 2 x SSC; 10 x Denhardt solution (Fikoll 400 + PEG + BSA; ratio 1:1:1); 0.1% SDS; 5 mM EDTA; 50 mM Na2HPO4; |
| | 250 µg/ml of herring sperm DNA; 50 µg/ml of tRNA; or |
| | 0.25 M of sodium phosphate buffer, pH 7.2; |
| | 1 mM EDTA |
| | 7% SDS |
| Hybridization temperature T | = 60°C |
| Washing buffer: | 2 x SSC; 0.1% SDS |
| Washing temperature T | = 60°C. |

Nucleic acid molecules which hybridize with the nucleic acid molecules of the invention can, in principle, encode a protein according to the invention from any organism expressing such proteins or can encode modified versions thereof.
Nucleic acid molecules which hybridize with the molecules of the invention can for instance be isolated from genomic libraries or cDNA libraries of bacteria, fungi, plants or animals. Preferably, such molecules are from animal origin, particularly preferred from human origin. Alternatively, they can be prepared by genetic engineering or chemical synthesis.
Such nucleic acid molecules may be identified and isolated by using the molecules of the invention or parts of these molecules or reverse complements of these molecules, for instance by hybridization according to standard methods (see for instance Sambrook et al., 1989, Molecular Cloning. A Laboratory Manual, 2nd edition, Cold Spring Harbor Laboratory Press, Cold Spring Harbor, NY).
Nucleic acid molecules comprising the same or substantially the same nucleotide sequence as indicated in Seq ID No: 1 or parts thereof can, for instance, be used as hybridization probes. The fragments used as hybridization probes can also be synthetic fragments which are prepared by usual synthesis techniques, and the sequence of which is substantially identical with that of a nucleic acid molecule according to the invention.
The molecules hybridizing with the nucleic acid molecules of the invention also comprise fragments, derivatives and allelic variants of the above-described nucleic acid molecules encoding a protein according to the invention. Herein, fragments are understood to mean parts of the nucleic acid molecules which are long enough to encode the described protein, preferably showing the biological activity of a protein according to the invention described above, e.g. being capable of interacting with the chemokine receptor CCR5 or other chemokine receptor family members. In this context, the term derivative means that the sequences of these molecules differ from the sequences of the above-described nucleic acid molecules in one or more positions and show a high degree of homology to these sequences. In this context, homology means a sequence identity of at least 40%, in particular an identity of at least 60%, preferably of more than 65%, even more preferably of at least 70%, in particular of at least 80%, more preferably of at least 90% and particularly preferred of more than 95%. Deviations from the above-described nucleic acid molecules may have been produced, e.g., by deletion, substitution, insertion and/or recombination. Preferably, the degree of homology is determined by comparing the respective sequence with the nucleotide sequence of the coding region of SEQ ID No: 1. When the sequences which are compared do not have the same length, the degree of homology preferably refers to the percentage of nucleotide residues in the shorter sequence which are identical to nucleotide residues in the longer sequence. The degree of homology can be determined conventionally using known computer programs such as the DNAstar program with the Clustalw analysis. This program can be obtained from DNASTAR, Inc., 1228 South Park Street, Madison, WI 53715 or from DNASTAR, Ltd., Abacus House, West Ealing, London W13 OAS UK (support@dnastar.com) and is accessible at the server of the EMBL outstation.
When using the Clustal analysis method to determine whether a particular sequence is, for instance, 80% identical to a reference sequence the settings are preferably as follows: PAIRGAP: 0.05; MATRIX: blosum; GAP OPEN: 10; END GAPS: 10; GAP EXTENSION: 0.05; GAP DISTANCE: 0.05; KTUP: 1; WINDOW LENGTH: 0; TOPDIAG: 1.
Furthermore, homology means preferably that the encoded protein displays a sequence identity of at least 50%, more preferably of at least 60%, even more preferably of at least 65%, in particular of at least 70%, particularly preferred of at least 80%, especially preferred of at least 90% and even more preferred of at least 95% to the amino acid sequence depicted under SEQ ID NO: 2.
Preferably, sequences hybridizing to a nucleic acid molecule according to the invention comprise a region of homology of at least 90%, preferably of at least 93%, more preferably of at least 95%, still more preferably of at least 98% and particularly preferred of at least 99% identity to an above-described nucleic acid molecule, wherein this region of homology has a length of at least 500 nucleotides, more preferably of at least 750 nucleotides, even more preferably of at least 800 nucleotides, particularly preferred of at least 900 nucleotides and most preferably of at least 1000 nucleotides.
Homology, moreover, means that there is a functional and/or structural equivalence between the corresponding nucleic acid molecules or proteins encoded thereby. Nucleic acid molecules which are homologous to the above-described molecules and represent derivatives of these molecules are normally variations of these molecules which represent modifications having the same biological function. They may be either naturally occurring variations, for instance sequences from other ecotypes, varieties, species, etc., or mutations, and said mutations may have formed naturally or may have been produced by deliberate mutagenesis. Furthermore, the variations may be synthetically produced sequences. The allelic variants may be naturally occurring variants or synthetically produced variants or variants produced by recombinant DNA techniques.
The proteins encoded by the different variants of the nucleic acid molecules of the invention possess certain characteristics they have in common. These include for instance biological activity, molecular weight, immunological reactivity, conformation, etc., and physical properties, such as for instance the migration behavior in gel electrophoreses, chromatographic behavior, sedimentation coefficients, solubility, spectroscopic properties, stability, pH optimum, temperature optimum etc.

The biological activity of the protein encoded by a nucleic acid molecule according to the invention, in particular the capacity to interact with the CCR5 receptor or other chemokine receptor family members, can be tested as described, e.g., in the Examples. In particular, it can be tested by a yeast two-hybrid system as described in Example 1 or by co-immunoprecipitation as described in Example 4.

In a particularly preferred embodiment, the nucleic acid molecule comprises a coding region which is not 100% identical to the coding region of SEQ ID NO:1, preferably a coding region which is not more than 99.2% identical to the coding region of SEQ ID NO:1, more preferably a coding region which is not more than 99% identical to the coding region of SEQ ID NO:1 and even more preferably a coding region which is not more than 98% identical to the coding region of SEQ ID NO:1.

The nucleic acid molecules of the invention can be DNA molecules, in particular genomic DNA or cDNA. Moreover, the nucleic acid molecules of the invention may be RNA molecules. The nucleic acid molecules of the invention can be obtained for instance from natural sources or may be produced synthetically or by recombinant techniques, such as PCR.

The nucleic acid molecules of the invention now allow host cells to be prepared which produce recombinant proteins encoded by these nucleic acid molecules of high purity and/or in sufficient quantities, as well as genetically engineered cells possessing an increased or reduced activity of these proteins.

The invention also relates to oligonucleotides specifically hybridizing to a nucleic acid molecule of the invention. Such oligonucleotides have a length of preferably at least 10, in particular at least 15, and particularly preferably of at least 50 nucleotides. They are characterized in that they specifically hybridize to the nucleic acid molecules of the invention, that is to say that they do not or only to a very minor extent hybridize to nucleic acid sequences encoding other proteins. The oligonucleotides of the invention can be used for instance as primers for amplification techniques such as the PCR reaction or as a hybridization probe to isolate related genes.

Moreover, the invention relates to vectors, in particular plasmids, cosmids, viruses, bacteriophages and other vectors commonly used in genetic engineering, which contain the above-described nucleic acid molecules of the invention. In a preferred embodiment of the invention, the vectors of the invention are suitable for the transformation of fungal cells, cells of microorganisms or animal cells, in particular mammalian cells. Preferably, such vectors are suitable for the transformation of human cells. In a particularly preferred embodiment such vectors are suitable for gene therapy purposes.

In another preferred embodiment, the nucleic acid molecules contained in the vectors are connected to regulatory elements ensuring transcription and synthesis of a translatable RNA in prokaryotic or eukaryotic cells.
The expression of the nucleic acid molecules of the invention in prokaryotic or eukaryotic cells, for instance in Escherichia coli, is interesting because it permits a more precise characterization of the biological activities of the proteins encoded by these molecules. Moreover, it is possible to express these proteins in such prokaryotic or eukaryotic cells which are free from interfering proteins. In addition, it is possible to insert different mutations into the nucleic acid molecules by methods usual in molecular biology (see for instance Sambrook et al., 1989, Molecular Cloning, A Laboratory Manual, 2nd edition, Cold Spring Harbor Laboratory Press, Cold Spring Harbor, NY), leading to the synthesis of proteins possibly having modified biological properties. In this regard it is on the one hand possible to produce deletion mutants in which nucleic acid molecules are produced by progressive deletions from the 5' or 3' end of the coding DNA sequence, and said nucleic acid molecules lead to the synthesis of correspondingly shortened proteins.
On the other hand, the introduction of point mutations is also conceivable at positions at which a modification of the amino acid sequence for instance influences the biological activity or the regulation of the protein.
Moreover, mutants possessing a modified substrate or product specificity can be prepared. Furthermore, it is possible to prepare mutants having a modified activity-temperature-profile.

Furthermore, in the case of expression in human cells, the introduction of mutations into the nucleic acid molecules of the invention allows the gene expression rate and/or the activity of the proteins encoded by the nucleic acid molecules of the invention to be reduced or increased.
For genetic engineering in prokaryotic cells, the nucleic acid molecules of the invention or parts of these molecules can be introduced into plasmids which permit mutagenesis or sequence modification by recombination of DNA sequences. Standard methods (see Sambrook et al., 1989, Molecular Cloning: A laboratory manual, 2nd edition, Cold Spring Harbor Laboratory Press, NY, USA) allow base exchanges to be performed or natural or synthetic sequences to be added. DNA fragments can be connected to each other by applying adapters and linkers to the fragments. Moreover, engineering measures which provide suitable restriction sites or remove surplus DNA or restriction sites can be used. In those cases, in which insertions, deletions or substitutions are possible, in vitro mutagenesis, "primer repair", restriction or ligation can be used. In general, a sequence analysis, restriction analysis and other methods of biochemistry and molecular biology are carried out as analysis methods.

Another embodiment of the invention relates to host cells, in particular prokaryotic or eukaryotic cells transformed and/or genetically modified with an above-described nucleic acid molecule of the invention or with a vector of the invention, and to cells derived from such transformed cells and containing a nucleic acid molecule or vector of the invention. In a preferred embodiment the host cell is genetically modified in such a way that it contains a nucleic acid molecule stably integrated into the genome. More preferably the nucleic acid molecule can be expressed so as to lead to the production of a protein having the biological activity of an AIP protein.
An overview of different expression systems is for instance contained in Methods in Enzymology 153 (1987), 385-516, in Bitter et al. (Methods in Enzymology 153 (1987), 516-544) and in Sawers et al. (Applied Microbiology and Biotechnology 46 (1996), 1-9), Billman-Jacobe (Current Opinion in Biotechnology 7 (1996), 500-4), Hockney (Trends in Biotechnology 12 (1994), 456-463), Griffiths et al., (Methods in Molecular Biology 75 (1997), 427-440). An overview of yeast expression systems is for instance given by Hensing et al. (Antonie van Leuwenhoek 67 (1995), 261-279), Bussineau et al. (Developments in Biological Standardization 83 (1994), 13-19), Gellissen et al. (Antonie van Leuwenhoek 62 (1992), 79-93, Fleer (Current Opinion in Biotechnology 3 (1992), 486-496), Vedvick (Current Opinion in Biotechnology 2 (1991), 742-745) and Buckholz (Bio/Technology 9 (1991), 1067-1072).
Expression vectors have been widely described in the literature. As a rule, they contain not only a selection marker gene and a replication-origin ensuring replication in the host selected, but also a bacterial or viral promoter, and in most cases a termination signal for transcription. Between the promoter and the termination signal there is in general at least one restriction site or a polylinker which enables the insertion of a coding DNA sequence. The DNA sequence naturally controlling the transcription of the corresponding gene can be used as the promoter sequence, if it is active in the selected host organism. However, this sequence can also be exchanged for other promoter sequences. It is possible to use promoters ensuring constitutive expression of the gene and inducible promoters which permit a deliberate control of the expression of the gene. Bacterial and viral promoter sequences possessing these properties are described in detail in the literature. Regulatory sequences for the expression in microorganisms (for instance E. coli, S. cerevisiae) are sufficiently described in the literature. Promoters permitting a particularly high expression of a downstream sequence are for instance the T7 promoter (Studier et al., Methods in Enzymology 185 (1990), 60-89), lacUV5, trp, trp-IacUV5 (DeBoer et al., in Rodriguez and Chamberlin (Eds), Promoters, Structure and Function; Praeger, New York, (1982), 462-481; DeBoer et al., Proc. Natl. Acad. Sci. USA (1983), 21-25), Ip1, rac (Boros et al., Gene 42 (1986), 97-100). Inducible promoters are preferably used for the synthesis of proteins. These promoters often lead to higher protein yields than do constitutive promoters. In order to obtain an optimum amount of protein, a two-stage process is often used. First, the host cells are cultured under optimum conditions up to a relatively high cell density. In the second step, transcription is induced depending on the type of promoter used. In this regard, a tac promoter is particularly suitable which can be induced by lactose or IPTG (=isopropyl-β-D-thiogalactopyranoside) (deBoer et al., Proc. Natl. Acad. Sci. USA 80 (1983), 21-25). Termination signals for transcription are also described in the literature.
The transformation of the host cell with a nucleic acid molecule or vector according to the invention can be carried out by standard methods, as for instance described in Sambrook et al., (Molecular Cloning: A Laboratory Manual, 2nd edition (1989) Cold Spring Harbor Press, New York; Methods in Yeast Genetics, A Laboratory Course Manual, Cold Spring Harbor Laboratory Press, 1990). The host cell is cultured in nutrient media meeting the requirements of the particular host cell used, in particular in respect of the pH value, temperature, salt concentration, aeration, antibiotics, vitamins, trace elements etc. The protein according to the present invention can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the protein. Finally, high performance liquid chromatography (HPLC) can be employed for final purification steps.

Moreover, the invention relates to a protein and biologically active fragments thereof, which is encoded by a nucleic acid molecule according to the invention and to methods for its preparation, wherein a host cell according to the invention is cultured under conditions permitting the synthesis of the protein, and the protein is subsequently isolated from the cultured cells and/or the culture medium. The present invention also relates to the protein obtainable by such a method. The protein of the present invention may, e.g., be a naturally purified product, or a product of chemical synthetic procedures, or produced by recombinant techniques from a prokaryotic or eukaroytic host (for example, by bacterial, yeast, higher plant, insect and mammalian cells in culture). Depending upon the host employed in a recombinant production procedure, the protein of the presen invention may be glycosylated or may be non-glycosylated. The protein of the invention may also include an initial methionine amino acid residue. The protein according to the invention may be further modified to contain additional chemical moieties not normally part of the protein. Those derivatized moieties may, e.g., improve the stability, solubility, the biological half life or absorption of the protein. The moieties may also reduce or eliminate any undesirable side effects of the protein and the like. An overview for these moieties can be found, e.g., in Remington's Pharmaceutical Sciences (18th ed., Mack Publishing Co., Easton, PA (1990)). Polyethylene glycol (PEG) is an example for such a chemical moiety which has been used for the preparation of therapeutic proteins. The attachment of PEG to proteins has been shown to protect them against proteolysis (Sada et al., J. Fermentation Bioengineering 71 (1991), 137-139). Various methods are available for the attachment of certain PEG moieties to proteins (for review see: Abuchowski et al., in "Enzymes as Drugs"; Holcerberg and Roberts, eds. (1981), 367-383). Generally, PEG molecules are connected to the protein via a reactive group found on the protein. Amino groups, e.g. on lysines or the amino terminus of the protein are convenient for this attachment among others.

Preferably, the protein according to the invention comprises an amino acid sequence which is not 100% identical to the amino acid sequence shown in SEQ ID NO:2. In particular, it is preferred that the protein has an amino acid sequence which is not more than 99.5%, more preferred not more than 99% and even more preferred not more than 98% identical to the amino acid sequence shown in SEQ ID NO:2.

The present invention also relates to mutants of a protein of the invention, in which the mutation is such that the region responsible for interaction with the CCR5 receptor is modified in such a way that the protein can no longer interact with CCR5. Such a mutation is preferably an amino acid substitution, deletion or insertion in the region responsible for interaction with the CCR5 receptor. More preferably the mutation is located in the region corresponding to amino acid residues 139 to 178 shown in SEQ ID NO:2, most preferably the mutation destroys the hydrophobic pocket which is built by amino acid residues corresponding to amino acid residues 155 to 158 in SEQ ID NO:2. In a particularly preferred embodiment the mutation is the change of the amino acid residue corresponding to G157 in SEQ ID NO:2 to R (G157R).
Moreover, such a mutant protein preferably has the property of being able to decrease or inhibit HIV infection, in particular when tested as described in Example 5.

Furthermore, the present invention also relates to an antibody specifically recognizing a protein according to the invention. The antibody can be monoclonal or polyclonal and can be prepared according to methods well known in the art. The term "antibody" also comprises fragments of an antibody which still retain the binding specificity.
The protein according to the invention, its fragments or other derivatives thereof, or cells expressing them can be used as an immunogen to produce antibodies thereto. The present invention in particular also includes chimeric, single chain, and humanized antibodies, as well as Fab fragments, or the product of an Fab expression library. Various procedures known in the art may be used for the production of such antibodies and fragments.
Antibodies directed against a protein according to the present invention can be obtained, e.g., by direct injection of the protein into an animal or by administering the protein to an animal, preferably a non-human animal. The antibody so obtained will then bind the protein itself. In this manner, even a sequence encoding only a fragment of the protein can be used to generate antibodies binding the whole native protein. Such antibodies can then, e.g., be used to isolate the protein from tissue expressing that polypeptide or to detect it in a probe. For the preparation of monoclonal antibodies, any technique which provides antibodies produced by continuous cell line cultures can be used. Examples for such techniques include the hybridoma technique (Köhler and Milstein, Nature 256 (1975), 495-497), the trioma technique, the human B-cell hybridoma technique (Kozbor et al., Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Techniques describing the production of single chain antibodies (e.g., U.S. Patent 4,946,778) can be adapted to produce single chain antibodies to immunogenic proteins according to the present invention. Furthermore, transgenic mice may be used to express humanized antibodies directed against immunogenic proteins of the present invention.
In a preferred embodiment the antibody is an antibody which, when binding to the protein according to the invention, inhibits the interaction of the protein with the CCR5 receptor or other chemokine receptor family members.

The present invention also relates to a pharmaceutical composition comprising a protein or mutant thereof according to the invention or a nucleic acid molecule which is suitable to express such a protein or mutant in target cells, and optionally a pharmaceutically acceptable carrier or excipient.

Moreover, the present invention also relates to the use of a protein or of a mutant protein according to the invention or of a nucleic acid molecule which is suitable to express such a protein in target cells for the preparation of a pharmaceutical composition for the prevention and/or treatment of HIV infection and/or progression. The term "HIV" in the context of the present invention refers to all types and variants of HIV, in particular to HIV1 and HIV2. Both types are known to use the CCR5 receptor as co-receptor (Reeves et al., J. Virol. 73 (1999), 7795-7804; Morner et al., J. Virol. 73 (1999), 2343-2349).

The pharmaceutical compositions according to the invention may be suitable to be administered orally, rectally, parenterally, intracisternally, intradermally, intravaginally, intraperitoneally, topically (as by powders, ointments, gels, creams, drops or transdermal patch), bucally, or as an oral or nasal spray. By "pharmaceutically acceptable carrier or excipient" is meant a non-toxic solid, semisolid or liquid filter, diluent, encapsulating material or formulation auxiliary of any type. The term "parenteral" as used herein refers to modes of administration which include intravenous, intramuscular, intraperitoneal, intrasternal, subcutaneous and intraarticular injection and infusion. It is understood that, when administered to a human patient, the total daily usage of the pharmaceutical compositions of the present invention will be decided by the attending physician within the scope of sound medical judgement. The specific therapeutically effective dose level for any particular patient will naturally depend upon a variety of factors including the age, body weight, general health, sex and diet of the patient; the time of administration, route of administration, and rate of excretion of the composition; the duration of the treatment; drugs (such as a chemotherapeutic agent) used in combination or coincidental with the specific composition and the like. Suitable formulations can be found, e.g., in Remington's Pharmaceutical Sciences, Mack Publishing Company, Easton, PA.
The protein according to the present invention may also be employed by expression of such a protein in vivo, which is also referred to as "gene therapy".

Thus, for example, cells from a patient may be engineered with a polynucleotide (DNA or RNA) encoding a protein ex vivo, with the engineered cells then being provided to a patient to be treated with the protein. Such methods are well known in the art. For example, cells may be engineered by procedures known in the art by use of a retroviral particle containing RNA encoding a protein according to the present invention.

The present invention also relates to an antagonist of the protein according to the invention. "Antagonist" in this regard preferably means that such a compound reduces or prevents the interaction of a protein according to the invention and a CCR5 receptor or another transmembrane receptor, preferably another chemokine receptor family member. In this context and in the following the term "transmembrane receptor" refers to a transmembrane receptor as defined herein-above. This can, e.g., be effected by a reduction of the amount of a protein according to the invention in cells, by blocking its binding site for CCR5 or another transmembrane receptor, preferably another chemokine receptor family member, or by blocking the C-terminus of CCR5. Thus, examples of antagonists are an antibody according to the invention, preferably an antibody the binding of which interferes with the interaction of the protein of the invention with a CCR5 receptor or another transmembrane receptor, preferably another chemokine receptor family member, or an antisense construct directed against a transcript of a nucleic acid molecule according to the invention or a nucleotide sequence encoding such an antisense construct.
The antisense approach is an approach well known in the art and it can be used to control gene expression through triple-helix formation or antisense DNA or RNA, both of which methods are based on binding of a polynucleotide to DNA or RNA. For example, the 5' coding portion of a nucleotide sequence which codes for the protein of the present invention is used to design an antisense RNA oligonucleotide of from about 10 to 40 base pairs in length. The antisense RNA oligonucleotide hybridizes to the corresponding DNA in vivo and blocks translation of the DNA molecule. The antisense oligonucleotides described above can be delivered directly into cells or corresponding nucleotide sequences may be delivered into the cells such that the antisense RNA or DNA may be expressed in vivo.

Another example for an antagonist is a sequence encoding a fragment of the protein according to the invention which is active as a dominant negative mutant. Such fragments are in particular fragments which comprise the region which is responsible for the interaction with CCR5 or another transmembrane receptor, preferably another chemokine receptor family member, in particular the region corresponding to amino acid residues 139 to 178 of SEQ ID NO:2.
Further examples of antagonists are ribozymes which specifically cleave transcripts encoding a protein according to the invention or cosuppression RNAs which, upon expression in a cell, lead to a cosuppression effect. The construction and use of ribozymes and of cosuppression RNAs is known to the person skilled in the art.
The term "antagonist" as used herein also comprises DNA molecules encoding the above-described antisense RNAs, ribozymes or cosuppression RNAs.

Moreover, the term "antagonist" in particular, relates to compounds which possess the ability to block or disrupt the interaction between the CCR5 receptor or another transmembrane receptor, preferably another chemokine receptor family member, and the protein according to the invention. Examples for such compounds are fragments derived form the polypeptide according to the invention such as the cytoplasmatic domain or a fragment representing the hydrophobic pocket or mutants with hyperbinding activity (e.g. raised by molecular evolution).
Other examples for such compounds are, e.g. CCR5-derived peptides, that mimic the C-terminus of CCR5 and bind to the protein according to the invention, thereby preventing the association with endogenous CCR5 receptors. In vivo, these peptides, e.g., can be identified by expression in a suitable host cell system via retroviral vectors. Immunoprecipitation of the CCR5 receptor as described in Example 4 could then be used to reveal whether the protein according to the invention is coprecipitating. If a given peptide blocks the association, no protein according to the invention is detectable.
Subsequently, these CCR5-derived peptides or fragments derived from the protein according to the invention can be expressed in cells susceptible to HIV infection. Cells are infected with HIV as described in Example 5 and the effect of the expressed CCR5-derived peptides or fragments derived from the protein of the invention can be analyzed as in Example 5. Any CCR5-derived peptides or fragments derived from the protein of the invention that disrupt binding of CCR5 to the protein of the invention would block HIV infection.

The invention also relates to small molecules that are able to disrupt the interaction of CCR5 or another transmembrane receptor, preferably another chemokine receptor family member, and the protein of the invention. These compounds can be identified, e.g. in an ELISA based interaction system with recombinant CCR5 or another transmembrane receptor, preferably another chemokine receptor family member, and recombinant protein of the invention. One of the proteins can be immobilized and subsequently be incubated with the other recombinant protein with or without the substance of interest. If a substance blocks the interaction between the two molecules no signal can be detected. Afterwards, these molecules can be tested in an HIV infection system as described above and should inhibit HIV infection.
Such peptides can in particular be peptides comprising the sequence TGEQEISVGL of the CCR5 receptor. Moreover, antagonists can also be peptide mimetics, i.e. chemical compounds with similar principal properties as an above described peptide thereby mimicking its interactions.

Most preferably, an antagonist according to the invention has the ability to decrease or inhibit infection of cells with HIV, in particular when tested as described in Example 5.

The present invention also relates to methods for identifying a compound which can act as an antagonist of a protein according to the invention, preferably in so far as it is capable of interfering with the interaction of a protein of the invention with a CCR5 receptor or another transmembrane receptor, preferably another chemokine receptor family member, preferably being capable of disrupting said interaction, characterized by the following steps:
(a) expression of a CCR5 receptor or another transmembrane receptor, preferably another chemokine receptor family member, and of a protein according to the invention in a suitable host cell;
(b) immunoprecipitation of said receptor or of the protein of the invention in the presence of the compound to be tested; and
(c) determination of whether the protein of the invention or said receptor, respectively, was co-precipitated,
wherein the observation that no co-precipitation of the protein of the invention or of the said receptor, respectively, occurs is indicative of the property of the compound to block or disrupt the interaction between the receptor and the protein of the invention.

The present invention also relates to methods for identifying a compound which can act as an antagonist of the protein of the invention, in particular, in the above mentioned sense, which are ELISA based interaction systems and in which either a receptor as defined above, in particular a CCR5 receptor, or a protein of the invention is immobilized and is subsequently incubated with the respective other protein (i.e. a protein of the invention or the receptor) and a compound to be tested under conditions normally allowing interaction between a receptor as defined above, in particular a CCR5 receptor, and a protein of the invention, and wherein it is subsequently determined whether the compound blocks or disrupts the interaction between the receptor and the protein of the invention. If no signal is detected, this is indicative that the compound is capable of blocking or disrupting the interaction. As a control experiment the same assay is carried out in which no compound is added.

The present invention also relates to a pharmaceutical composition comprising an antagonist of the protein according to the invention and optionally a pharmaceutically acceptable carrier or excipient.

The present invention furthermore relates to the use of an antagonist of the protein according to the invention for the preparation of a pharmaceutical composition for the prevention or treatment of HIV infection and/or progression.

Furthermore, the present invention relates to a method for preparing a pharmaceutical composition comprising the steps of identifying an antagonist of the protein according to the invention by one of the methods described above and formulating the identified antagonist in a pharmaceutical composition. For the formulation of the pharmaceutical composition the same applies as described above for the pharmaceutical compositions according to the invention.

Moreover, the present invention relates to a diagnostic kit or composition comprising the protein, the oligonucleotide, the nucleic molecule or the antibody according to the invention.
The oligonucleotide or the nucleic acid molecule according to the invention may, e.g., be used for determining whether an individual carries a mutation in the gene encoding the protein according to the invention, thereby allowing to determine whether the individual suffers from a disease or has the susceptibility for a disease related to the presence of mutations in the gene.
A variety of techniques is available to detect mutations at the DNA level. For example, nucleic acids for diagnosis may be obtained from a patient's cells, such as from blood, urine, saliva, tissue biopsy and autopsy material. The genomic DNA may be used directly for detection or may be amplified enzymatically by using PCR prior to analysis. RNA or cDNA may also be used for the same purpose. As an example, PCR primers complementary to the nucleic acid encoding the protein according to the invention can be used to identify and analyze mutations. Deletions and insertions can, e.g., be detected by a change in size of the amplified product in comparison to the normal genotype. Point mutations can be identified by hybridizing amplified DNA to radiolabeled RNA encoding the protein according to the invention or alternatively, radiolabeled antisense DNA sequences. Perfectly matched sequences can be distinguished from mismatched duplexes by RNase A digestion or by differences in melting temperatures.
Genetic testing based on DNA sequence differences may be achieved by detection of alteration in electrophoretic mobility of DNA fragments in gels with or without denaturing agents. Small sequence deletions and insertions can be visualized by high resolution gel electrophoresis. DNA fragments of different sequences may be distinguished on denaturing formamide gradient gels in which the mobilities of different DNA fragments are retarded in the gel at different positions according to their specific melting or partial melting temperature.
The protein and/or the antibody according to the present invention can, e.g., be used in a diagnostic assay for detecting altered levels of a protein according to the invention in various tissues. An over- or under-expression of the protein compared to normal control tissue samples may detect the presence of a disease or susceptibility to a disease. Suitable assays are well known in the art and include radioimmunoassays, competitive-binding assays, Western Blot analysis, ELISA assays and "sandwich" assay.

The present invention also relates to the use of a protein according to the invention for the identification of compounds which may be of interest as pharmaceuticals, preferably for the identification of compounds which act as antagonists of the protein of the invention.

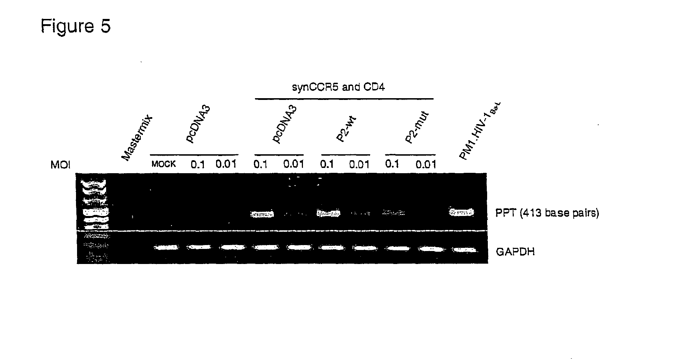

These and other embodiments are disclosed and obvious to a skilled person and embraced by the description and the examples of the present invention. Additional literature regarding one of the above-mentioned methods, means and applications, which can be used within the meaning of the present invention, can be obtained from the state of the art, for instance from public libraries for instance by the use of electronic means. This purpose can be served inter alia by public databases, such as the "medline", which are accessible via internet, for instance under the address http://www.ncbi.nlm.nih.gov/PubMed/medline.html. Other databases and addresses are known to a skilled person and can be obtained from the internet, for instance under the address http://www.lycos.com. An overview of sources and information regarding patents and patent applications in biotechnology is contained in Berks, TIBTECH 12 (1994), 352-364.
All of the above cited disclosures of patents, publications and database entries are specifically incorporated herein by reference in their entirety to the same extent as if each such individual patent, publication or entry were specifically and individually indicated to be incorporated by reference.

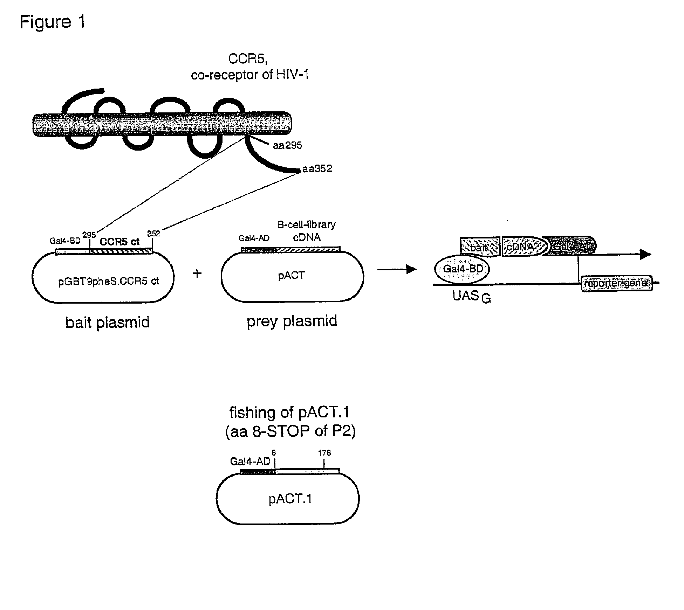

- **Figure 1**: shows schematically a yeast two-hybrid screen with the carboxyterminus of CCR5 and identification of a binding protein.
The seven-transmembrane protein CCR5 is shown schematically. Its carboxyterminus (aa295-352) which is deleted in LTNP, was used as a bait to screen for interacting proteins in the yeast two-hybrid system. A reporter assay allows the identification of an interaction between two proteins by blue yeast colonies. The carboxyterminus of CCR5 is fused to the Gal4-binding domain (BD) which binds to a specific DNA sequence. The prey proteins are encoded by a cDNA library from B-cells (prey). Interaction of CCR5 and prey protein that is fused to the Gal4-activation domain (AD), subsequently leads to the activation of the reporter gene.
One novel interaction partner (pACT.1) has been termed P2.

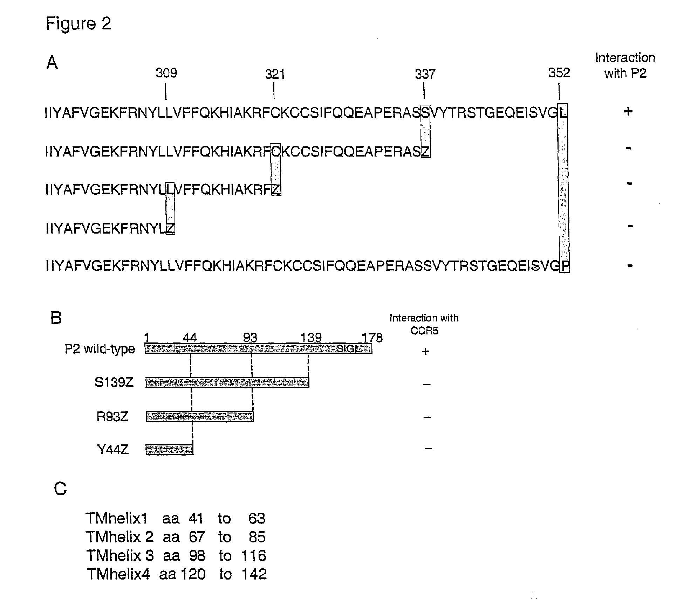

- **Figure 2**: shows the mapping of the site of interaction between the CCR5 receptor with P2.
**A.** The carboxyterminal amino acids of CCR5 that were used as a bait in the yeast two-hybrid screen are shown in the first line. Various deletion mutants are indicated below. These were assayed for interaction with P2 in the yeast two-hybrid screen; results are shown on the right.
**B.** Various deletion mutants of P2 were generated and tested for interaction with the CCR5 C-terminus in yeast. Results are shown on the right. Deletion of the putative binding motif in P2 (SIGL) destroys the binding to CCR5. Amino acids 155-158 (SIGL) form a hydrophobic pocket. A mutant was constructed to destroy this property by mutating G157 to R (G157R) used in Fig. 5.
**C.** The putative localization of transmembrane helices (TMhelix) as predicted with TMHMm1.0 (CBS, Denmark; http://www.cbs.dtu.dk/services/TMHMM-1.0).

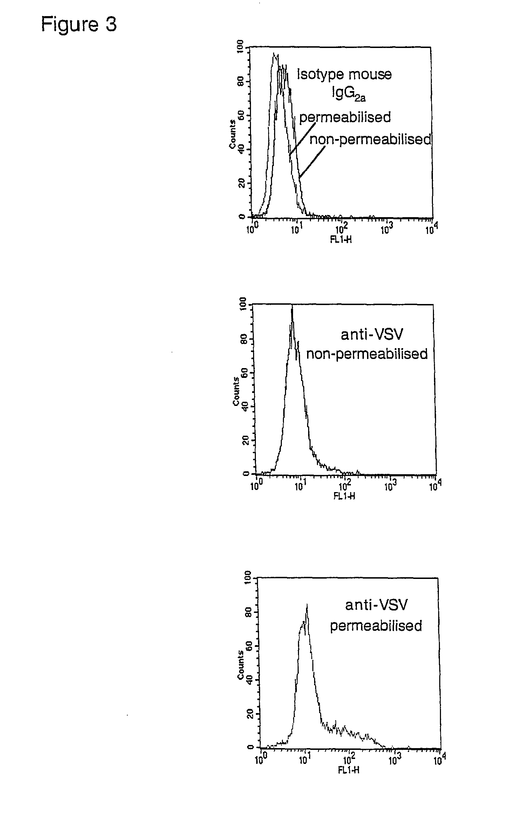

- **Figure 3**: shows the analysis of the orientation of P2 in the membrane.
The P2 protein was transiently transfected and expressed as a fusion protein with an aminoterminal VSV-tag (SIGMA Product: V5507, generated against the 15 carboxy terminal amino acids (497-511) of Vesicular Stomatitis Virus Glycoprotein) in HEK-293 cells. The cells were analysed by fluorescence-activated cell sorting (FACS) analysis with and without prior permeabilisation using Triton X-100 (0.5%) in physiological buffer. Unspecific binding of antibodies to the cells was monitored using mouse immunoglobulin G2a (IgG2a) isotype control (top). Mouse anti-VSV antibody was added to non-permeabilized (middle) and permeabilized cells (bottom). The scattered signal to the right of the peak indicate a positive signal for specific antibody binding to the VSV-tag. Only permeabilized cells show a positive signal indicating that the VSV-tagged aminoterminus is indeed intracellular.
- **Figure 4**: shows the analysis of interaction of CCR5 with P2 in mammalian cells.
The interaction between the HIV co-receptor CCR5 and P2 was verified in the mammalian cell-line Cf2Th stably expressing codon-optimized and carboxyterminally tagged with nine amino acids (C9), designated as synCCR5-C9 (Mirzabekov et al., J. Biol. Chem. 274 (1999), 28745-28750). Cells were transfected with plasmids encoding VSV-tagged P2 as indicated. After 36 hours the cells were fractioned into membrane and cytoplasm by ultracentrifugation. The interaction was demonstrated by co-immuno-precipitation. Antibodies (PharMingen Product: 36461A, clone 2D7 generated against the amino terminus of CCR5) were used to precipitate the receptor proteins. Interacting proteins that co-precipitated with the receptor were detected with antibodies against the VSV-tagged protein P2. Both, CCR5 and P2, are expressed in the membrane. The result shows the interaction of P2 and CCR5 as P2 co-precipitates with the CCR5 receptor.
Abbreviations used are: α for antibodies, DL for direct cell lysate, IP for immuno-precipitation.
- **Figure 5**: shows that a wild-type P2 (P2-wt) but not a mutant (P2-mut) allows HIV-1 infection as indicated by HIV specific PPT PCR product.
Abbreviations used are: MOI for multiplicities of infection, PPT for polypurine tract, GAPDH for glyceraldehyde-3-phosphate dehydrogenase.

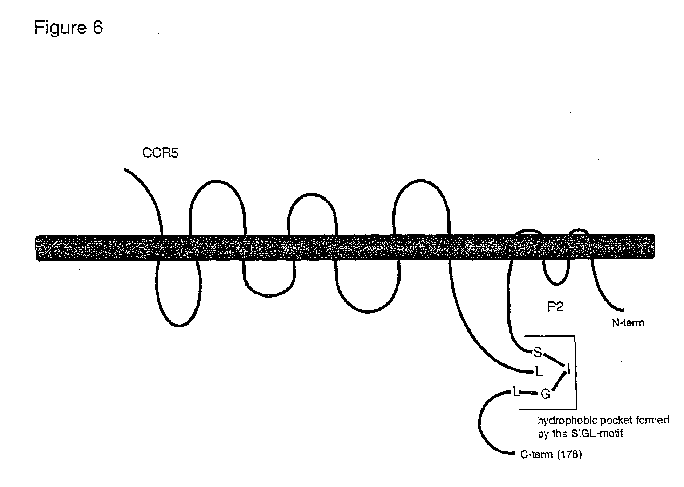

- **Figure 6**: shows a model for interaction of CCR5 and P2. The transmembrane protein P2 binds with its hydrophobic pocket reminescent of a PDZ-like domain (SIGL-motif) to the C-terminal leucine (aa352) of CCR5. In the yeast two-hybrid system deletion of the C-terminus of P2 including this motif abolishes binding to CCR5 (compare Fig. 2B). The SIGL motif was mutated to SIRL in order to destroy the hydrophobic pocket. This mutant interfered with HIV infection (compare Fig. 5). The four transmembrane helices are specified in Fig. 2C. The carboxyterminus of P2 is indicated (amino acid 178), the aminoterminus is shown to be cytoplasmic as evidenced in Fig. 3.

The following Examples serve to further illustrate the invention.

### Example 1

### Isolation of a nucleotide sequence encoding a chemokine receptor CCR5-interacting protein

Identification of interacting proteins by the yeast two-hybrid system is based upon the detection of the expression of a reporter gene, the transcription of which is dependent upon the reconstitution of a transcriptional regulator by the interaction of two proteins, each one fused to one half of the transcriptional regulator (Fig. 1). A given protein, called the bait, and a library of proteins, the prey, are expressed as fusion proteins to a DNA binding domain and a transcriptional regulatory domain, respectively.

In the present case, the DNA binding domain of the yeast transcriptional activator Gal4 is N-terminally fused to the CCR5 carboxyterminus CCR5 ct (amino acid 295 to 352) (plasmid pGBT9PheS-CCR5 ct, CLONTECH) and a human B cell cDNA library (Durfee et al., Genes & Dev. 7 (1993), 555-569) is fused to the activation domain of Gal4 (plasmid pACT, CLONTECH). Both plasmids are lithiumacetate-transformed (www.umanitoba homepage; in particular, www.umanitoba.ca/faculties/medicine/units/biochem/gietz/Trafo.html) into yeast strain Y153 that features a Gal4 dependent β-galactosidase and histidine expression (Durfee et al., loc. cit.). Transformants are plated and grown on minimal base medium (CLONTECH) lacking uracile (selection for Y153), tryptophane (selection for pGBT9PheS-CCR5 ct), leucine (selection for pACT) and histidine (selection for induction of Gal4 transcriptional activity). The medium also contains 7.5mM 3-amino-triazol in order to repress basal Gal4 transcription. When the colony size reaches 2-3 mm, lift assays are performed to screen for β-galactosidase expression. For this purpose, yeast colonies are overlaid with duralon membranes and the attached cells are shock-frozen twice in liquid nitrogen in order to break up cells and liberate β-galactosidase. After a brief thawing period, the membranes are placed on Whatman 3MM paper soaked with a 5-bromo-4-chloro-3-indolyl-b-D-galactopyranosid (X-gal, 0.5mg/ml) containing buffer. Due to the conversion of X-gal, the β-galactosidase positive colonies appear blue after 2-12 hours of incubation at room temperature. The corresponding colonies are picked from the original plates, amplified and plasmid DNA is extracted using a BIO 101 Fast DNA kit (BIO101). The isolated DNA is transformed into competent E.coli DH5α cells using standard techniques. Bacterial cultures are streaked out onto LB agar plates containing chlorophenylalanine (2g/L). The pGBT9PheS-HA-CCR5ct transformed DH5α cells do not survive due to a mutated phenylalanine-tRNA-synthetase subunit, but only pACT harbouring cells are selected. Plasmids are conventionally isolated and are reintroduced together with pGBT9PheS-HA-CCR5ct into Y153 via lithiumacetate transformation. Growing colonies are screened again for β-galactosidase activity.
Of initially 20 clones, one scored repeatedly positive after retransformation. The nucleotide sequence of this clone was determined using standard methods and is shown in SEQ ID NO: 1. The deduced amino acid sequence is shown in SEQ ID NO:2. The location of putative transmembrane domains of the encoded protein are shown in Figure 2C. In the context of the present invention the protein will be referred to as P2.

### Example 2

### Interaction of CCR5 with P2 analyzed by in vitro mutagenesis

The protein P2 was detected as interaction partner with CCR5 that proved to be specific after several controls. The respective cDNA clone was sequenced and further analyzed via data base. Based on that information the clone was generated in order to characterize the sites of interaction of P2 with the CCR5 receptor. A series of deletion mutants was generated to narrow down the specific region (Fig. 2A). The results show that for the interaction of CCR5 with the P2 protein the very carboxyterminal leucine (L352) of CCR5 seems to be essential, as mutation of this single leucine to proline (L352P) abolishes the interaction, indicating the importance of the terminal amino acid.
P2 was further characterized by screening for homologous sequences deposited in data bases. The P2 protein is of unknown function but is related to genes in other organisms. It is a putative adaptor-like protein which appears to link the receptor to the cytoskeleton. Computer predictions on the biological functions of P2 indicate that it is a transmembrane protein with two to four transmembrane domains.
CCR5 and P2 were truncated by generating deletion mutants, as shown in Fig. 2A/B, revealing that the protein needs its carboxyterminal domain in order to bind to CCR5. The regions recognized on the receptor are indicated. The interaction of P2 with the CCR5 is mediated by the carboxyterminal leucine of CCR5 and is abolished by mutation of this leucine to proline (Fig. 2A) (Songyang et al., Science 275 (1997), 73). Several other proteins with terminal leucines or valines have been characterized at the institute of the inventors and demonstrated to be ligands of so-called PDZ-domain containing proteins (Schneider et al., Nature Biotech. 17 (1999), 170-175). PDZ-domain containing proteins have a hydrophobic pocket through which they bind other proteins. The sequence motif GLGF within this hydrophobic pocket interacts with the terminal valine, leucine or isoleucine of target proteins, thus mediating interaction and clustering of proteins (Ponting et al., Bioessays 19 (1997), 469). Sequence analysis of P2 revealed a motif in the carboxyterminal region that is remotely related to the GLGF sequence of many other PDZ-proteins. This sequence SIGL (aa155-158) is conserved in the protein of C.elegans with a homology to P2, which indicates a high degree of conservation. Deletion of the region containing this motif in P2 abolishes interaction with CCR5 (Fig. 2B).

A mutation was introduced into the SIGL-motif-containing domain in P2, G157R, and it was tested whether this leads to disruption of the interaction with CCR5. This would prove that P2 is a novel type of PDZ-like protein with a so far unknown hydrophobic pocket and lacking the canonical six beta sheets and two α-helices characteristic for PDZ proteins. The data-base comparison of the carboxyterminus of CCR5 with those of other chemokine receptors showed no homologies to PDZ-domains. The specificity of interaction between CCR5 and the putative PDZ-like domain-containing protein P2 could be demonstrated, proving that it is a PDZ-like domain (Fig. 2).
The P2 protein harbors one additional putative hydrophobic pocket (GIGL, amino acid 53-56), which is suggestive for a PDZ-like domain pocket. This GIGL was mutated (G53A) and did not have any influence on the binding to the CCR5 C-terminus (unpublished observation), indicating that it is not a PDZ-domain binding to CCR5. Binding to other receptor ligands is possible. PDZ-proteins often harbor more than one PDZ-domain which bind various ligands and lead to receptor clustering. Thus, it is considered that P2 is a novel subtype PDZ-domain.

### Example 3

### Orientation of the encoded protein in the membrane

Human embryonic kidney cells, HEK293, were transfected with pS'VSV-P2 (Fig. 3) expressing full-length P2. In order to allow its detection, P2 was fused to a VSV-tag, a sequence of the vesicula stomatitis virus, for which specific antibodies are available (SIGMA product: V5507, generated against the 15 carboxy terminal amino acids (497-511) of Vesicular Stomatitis Virus Glycoprotein). This was, e.g. also used in Kreis (EMBO J. 5 (1986), 931) and in Soldati and Perriard (Cell 66 (1991), 277). It was fused to the aminoterminus of P2 in order to determine whether it faces to the inside or outside of the cell. The transiently transfected cells were treated with detergent 0.5% Triton x-100 in physiological buffer for permeabilization and treated with non-specific immunoglobulin G2a (Fig. 3, top) of the specific anti-VSV antibodies (middle, bottom). Reduction of the peak size and formation of a shoulder to the right side of the peak indicates the interaction of the antibody with the VSV-tag. The result demonstrates that the aminoterminus is accessible only after permeabilization, meaning that it faces to the cytoplasmic region of cell membrane.

### Example 4

### Biochemical interaction between CCR5 and P2

The interaction of proteins in yeast cells needs to be verified in mammalian cells. For that purpose constructs used in yeast had to be recloned into mammalian expression vectors. A method to prove the interaction is a co-immunoprecipitation of the proteins. To control expression of P2 the plasmids were transfected in mammalian cells, followed by precipitation and immunological detection (data not shown). Due to their seven transmembrane regions, the chemokine receptor turned out to be difficult to solubilize for precipitation. Therefore, several cell-lines, detergents and methods were tested to establish a useful protocol for chemokine receptor purification. Best results were obtained when Cf2Th cells (canine thymocytes) stably expressing a codon-optimized CCR5 receptor, which is C-terminally tagged with nine amino acids (C9), designated as synCCR5-C9 (Mirzabekov et al., J. Biol. Chem. 274 (1999), 28745-28750), were fractioned into cytoplasm and membrane. Membrane proteins were solubilized in CymalTM-5 (0.5%) and the receptor was precipitated. Binding of P2 and CCR5 was demonstrated after transfection of Cf2Th cells stably expressing synCCR5 with P2 (Fig. 4).
These findings clearly demonstrate that the interaction of P2 with CCR5 is real. The functionality of the receptor was proven after transfection by demonstrating its internalization and signaling after stimulation with the natural chemokine ligand. Also a migration assay was set up that allowed to measure cellular chemotaxis (migration) along a chemokine gradient (data not shown).

### Example 5

### P2 has an influence on HIV infection

HEK-293 cells were transiently transfected with P2-wt or P2-mut DNA plasmids co-transfected with DNA encoding the codon-optimized synCCR5 and CD4 receptors as indicated (in this experiment the syn CCR5 rexeptor is not tagged with C9 as in Fig. 4, the C-terminal leucine is freely accessible). The P2-mut contains one amino acid exchange (G157R). About 36 hours later 1x106 cells were challenged with HIV-1Ba-L in different multiplicities of infections (MOI) for 6 hours. The nucleic acid was extracted with QIAamp RNA Blood Mini Kit, according to manufacturers instruction. The levels of HIV-1 viral nucleic acid were analysed with polymerase-chain reaction (PCR) using primers up- and downstream of the highly conserved HIV-1 Ba-L polypurine tract (PPT), situated in the nef gene. For positive control PCR was performed using lysates of PM1 cells (Lusso et al., J. Virol. 69 (1995), 3712-3720) infected with HIV-1Ba-L. The results of this experiment are shown in Fig. 5. PCR products generated with primers for glyceraldehyde-3-phosphate dehydrogenase (GAPDH) reflect that the amount of the eluated nucleic acids is constant (control). No HIV-1 PPT signals are detectable in the PCR master-mix and pcDNA3 transfected cells as PCR and empty vector control. For efficient HIV infection both co-receptors, CD4 and CCR5, have to be expressed. Neither vector control pcDNA3 nor P2-wt have an effect on HIV-1 infection while P2-mut interferes with infection since less viral nucleic acid is detectable. The level of HIV-1 infection as detected by PCR signal correlates with the MOI. The GAPDH control shows no variations in the amount of nucleic acids in the various lysates.

## Claims

1. A nucleic acid molecule encoding a protein interacting with the carboxy terminus of the chemokine receptor CCR5 or of other chemokine receptor family members selected from the group consisting of
(a) nucleic acid molecules encoding a protein which comprises the amino acid sequence indicated in Seq ID No:2;
(b) nucleic acid molecules comprising the nucleotide sequence of the coding region indicated in Seq ID No: 1;
(c) nucleic acid molecules encoding a protein, the amino acid sequence of which has a homology of at least 50% to the amino acid sequence indicated in Seq ID No: 2;
(d) nucleic acid molecules the complementary strand of which hybridizes to a nucleic acid molecule as defined in (a) or (b); and
(e) nucleic acid molecules, the nucleotide sequence of which deviates because of the degeneracy of the genetic code from the sequence of the nucleic acid molecules as defined in any one of (b), (c) or (d).

2. An oligonucleotide which specifically hybridizes with the nucleic acid molecule of claim 1.

3. A vector containing the nucleic acid molecule of claim 1.

4. The vector of claim 3, wherein the nucleic acid molecule is linked to regulatory elements ensuring transcription in eukaryotic and prokaryotic cells.

5. A host cell, which is genetically modified with a nucleic acid molecule of claim 1 or with a vector of claim 3 or 4.

6. A method for the production of a protein encoded by a nucleic acid molecule of claim 1 in which the host cell of claim 5 is cultivated under conditions allowing for the expression of the protein and in which the protein is isolated from the cells and/or the culture medium.

7. A protein encoded by the nucleic acid molecule of claim 1 or obtainable by the method of claim 6.

8. A mutant of the protein of claim 7, wherein the mutation leads to the loss of the capability of the protein to interact with the CCR5 receptor.

9. An antibody specifically recognizing the protein of claim 7.

10. A pharmaceutical composition comprising the protein of claim 7 or a nucleic acid molecule of claim 1.

11. Use of the protein of claim 7 or of the nucleic acid molecule of claim 1 for the preparation of a pharmaceutical composition for the prevention and/or treatment of HIV infection and/or progression.

12. An antagonist of the protein of claim 7.

13. A pharmaceutical composition comprising the antagonist of claim 12 or the mutant protein of claim 8.

14. Use of the antagonist of claim 12 or of the mutant protein of claim 8 for the preparation of a pharmaceutical composition for the prevention and/or treatment of HIV infection and/or progression.

15. A diagnostic composition comprising the nucleic acid molecule of claim 1, the oligonucleotide of claim 2, the protein of claim 7 and/or the antibody of claim 8.

16. A method for identifying a compound which can act as an antagonist of a protein of claim 7 in so far as it is capable of interfering with the interaction of the protein with a CCR5 receptor or another transmembrane receptor **characterized by** the following steps:
(a) expression of the receptor and of a protein of claim 7 in a suitable host cell;
(b) immunoprecipitation of said receptor or of said protein in the presence of the compound to be tested; and
(c) determination of whether said protein or said receptor, respectively, was co-precipitated,
wherein the observation that no co-precipitation of said protein or of the said receptor, respectively, occurs is indicative of the property of the compound to block or disrupt the interaction between the receptor and said protein.

17. A method for identifying a compound which can act as an antagonist of the protein of claim 7 in so far as it is capable of interfering with the interaction of said protein with a CCR5 receptor or another transmembrane receptor, wherein said method is an ELISA based interaction system in which either said receptor or said protein is immobilized and is subsequently incubated with the respective other protein (i.e. said protein or said receptor) and a compound to be tested under conditions normally allowing interaction between said receptor and said protein, and wherein it is subsequently determined whether the compound blocks or disrupts the interaction between said receptor and said protein, and wherein the absence of a signal is indicative that the compound is capable of blocking or disrupting the interaction.

18. A method for preparing a pharmaceutical composition comprising
(a) carrying out a method of claims 16 or 17; and
(b) formulating the antagonist identified according to (a) in a pharmaceutical composition.


SEQUENCE LISTING

<110> MOELLING, Karin
      Institut für medizinische Virologie der Universität Zürich

<120> Nucleic acid molecules encoding a protein interacting with the
      chemokine receptor CCR5 or other chemokine receptor family members

<130> E 2096 EP

<140>
<141>

<160> 2

<170> PatentIn Ver. 2.1

<210> 1
<211> 1263
<212> DNA
<213> Homo sapiens

<220>
<221> CDS
<222> (17)..(550)

<400> 1
cacattaacc ggcagg atg tcg gag gtg cgg ctg cca ccg cta cgc gcc ctg 52
                  Met Ser Glu Val Arg Leu Pro Pro Leu Arg Ala Leu
                    1               5                  10

gac gac ttt gtt ctg ggg tcg gcg cgt ctg gcg gct ccg gat cca tgc   100
Asp Asp Phe Val Leu Gly Ser Ala Arg Leu Ala Ala Pro Asp Pro Cys
         15                  20                  25

gac ccg cag cga tgg tgc cac cgc gtc atc aac aac ctc ctc tac tac   148
Asp Pro Gln Arg Trp Cys His Arg Val Ile Asn Asn Leu Leu Tyr Tyr
     30                  35                  40

caa acc aac tac ctt ctc tgc ttc ggc atc ggc ctc gct ctc gcc ggg   196
Gln Thr Asn Tyr Leu Leu Cys Phe Gly Ile Gly Leu Ala Leu Ala Gly
 45                  50                  55                  60

tac gtg cgg cca ctt cat acg ctc ctg agc gcg ctg gta gtg gcg gtg   244
Tyr Val Arg Pro Leu His Thr Leu Leu Ser Ala Leu Val Val Ala Val
                 65                  70                  75

gcc ctc ggc gtg ctg gtg tgg gca gct gag acc cgc gca gct gtg cgc   292
Ala Leu Gly Val Leu Val Trp Ala Ala Glu Thr Arg Ala Ala Val Arg
             80                  85                  90

cgc tgc cgc cgc agc cac cct gca gcc tgc ctg gcc gca gtg ctt gcc   340
Arg Cys Arg Arg Ser His Pro Ala Ala Cys Leu Ala Ala Val Leu Ala
         95                 100                 105

gtc ggc ctc ctg gtg ctc tgg gtc gcg ggc ggc gct tgc acc ttc ctg   388
Val Gly Leu Leu Val Leu Trp Val Ala Gly Gly Ala Cys Thr Phe Leu
    110                 115                 120

ttc agc atc gcc ggg ccg gtg ctt ctg atc ctg gtg cac gcc tcg ttg   436
Phe Ser Ile Ala Gly Pro Val Leu Leu Ile Leu Val His Ala Ser Leu
125                 130                 135                 140

cgc ctg cgc aac ctt aag aac aag att gag aac aag atc gag agc att   484

Arg Leu Arg Asn Leu Lys Asn Lys Ile Glu Asn Lys Ile Glu Ser Ile
                145                 150                 155

ggt ctc aag cgg acg cca atg ggc ctg cta cta gag gca ctg gga caa     532
Gly Leu Lys Arg Thr Pro Met Gly Leu Leu Leu Glu Ala Leu Gly Gln
            160                 165                 170

gag cag gag gct gga tcc taggcccctg ggatctgtac ccaggacctg           580
Glu Gln Glu Ala Gly Ser
        175

gagaatacca ccccaccccc agcccataat tgggacccag agccctttcc cagcacttaa 640

aacaggagcc tagagccccc tgcccaaaca aaacaggaca tctgtgaccg ccctaccccc 700

acgccagccc caaactaaga tatccctcac acccagcccc cattacctag ggacaagagt 760

cttccccagc cttgaaccta ggaccaagag ccacctacat ccagccccaa aactggggct 820

tcaggccaga gcatccatgg ccaatttcaa attgtgaacc cagagacact cccatccacc 880

cttctccatg ctcatcccca aactggggcc tggagcaagg cactctcaaa tcttgaaccc 940

tggaccaaag cttttccaga ccccacccta ccttccaacc caggtcaaga cattgccaaa 1000

tcttgaactc agaacccaag tgttccatgc ccctgtgtgg atggagtcgg gtatcctgac 1060

tgttggaccc ctggtccagg tgatcccgac cctcaccagt cccatttgcc tccctccagc 1120

tctgcttagg cattttgccc ctcaccccaa tgttccacac catcgacaac caaggggtga 1180

ggtggggaca ggcctcagca gggaatgggg cgtatatgtt agtgttgctg caacaataaa 1240

gcctgttgca tctctcatgc caa                                          1263

<210> 2
<211> 178
<212> PRT
<213> Homo sapiens

<400> 2
Met Ser Glu Val Arg Leu Pro Pro Leu Arg Ala Leu Asp Asp Phe Val
1               5                   10                  15

Leu Gly Ser Ala Arg Leu Ala Ala Pro Asp Pro Cys Asp Pro Gln Arg
            20                  25                  30

Trp Cys His Arg Val Ile Asn Asn Leu Leu Tyr Tyr Gln Thr Asn Tyr
        35                  40                  45

Leu Leu Cys Phe Gly Ile Gly Leu Ala Leu Ala Gly Tyr Val Arg Pro
    50                  55                  60

Leu His Thr Leu Leu Ser Ala Leu Val Val Ala Val Ala Leu Gly Val
65                  70                  75                  80

Leu Val Trp Ala Ala Glu Thr Arg Ala Ala Val Arg Arg Cys Arg Arg
                85                  90                  95

Ser His Pro Ala Ala Cys Leu Ala Ala Val Leu Ala Val Gly Leu Leu
            100                 105                 110

Val Leu Trp Val Ala Gly Gly Ala Cys Thr Phe Leu Phe Ser Ile Ala

115                 120                 125

Gly Pro Val Leu Leu Ile Leu Val His Ala Ser Leu Arg Leu Arg Asn
    130                 135                 140

Leu Lys Asn Lys Ile Glu Asn Lys Ile Glu Ser Ile Gly Leu Lys Arg
145                 150                 155                 160

Thr Pro Met Gly Leu Leu Leu Glu Ala Leu Gly Gln Glu Gln Glu Ala
                165                 170                 175

Gly Ser